(19) Europäisches Patentamt
European Patent Office
Office européen des brevets

(11) Publication number: 0 369 657 A2

(12) # EUROPEAN PATENT APPLICATION

(21) Application number: 89311426.4

(22) Date of filing: 03.11.89

(51) Int. Cl.5: C12Q 1/44, C12Q 1/34, G01N 33/58, //G01N33/535, G01N33/569,G01N33/571, C07C251/22

(30) Priority: 17.11.88 US 272561

(43) Date of publication of application: 23.05.90 Bulletin 90/21

(84) Designated Contracting States: AT BE CH DE ES FR GB GR IT LI LU NL SE

(71) Applicant: Becton Dickinson and Company
One Becton Drive
Franklin Lakes New Jersey 07417-1880(US)

(72) Inventor: Reardon, John E
7307 Calibre Park Drive
Aprt. 203 Durham North Carolina(US)

(74) Representative: Ruffles, Graham Keith et al
MARKS & CLERK 57-60 Lincoln's Inn Fields
London WC2A 3LS(GB)

(54) **Chromogenic substrate for esterase and immunoassay using same.**

(57) A chromogenic substrate for an esterase, 4-[(3,5-dichloro-4-butyryloxyphenyl)imino]-2,5-cyclohexadien-1-one, is useful in an assay for determination of the presence of concentration of an enzyme or a ligand in a liquid. The assay may be an ELISA using the substrate or a dual enzyme assay including a blocked inhibitor which is cleaved by a hydrolase to an inhibitor of the esterase. The invention includes a kit of materials including the substrate for performing an assay.

EP 0 369 657 A2

# CHROMOGENIC SUBSTRATE FOR ESTERASE AND IMMUNOASSAY USING SAME

## BACKGROUND OF THE INVENTION

### 1. Field of the Invention.

This invention relates to enzymes, and, more particularly, relates to a substrate for an esterase and use thereof in an assay.

### 2. Background.

Assay systems which are both rapid and sensitive have been developed to determine the concentration of a substance in a fluid. Immunoassays depend on the binding of an antigen or hapten to a specific antibody and have been particularly useful because they give high levels of specificity and sensitivity. These assays generally employ one of the above reagents in labeled form, the labeled reagent often being referred to as the tracer. Immunoassay procedures may be carried out in solution or on a solid support and may be either heterogeneous, requiring a separation of bound tracer from free (unbound) tracer or homogeneous in which a separation step is not required.

Enzymes have often been used as labels in immunoassay. In conventional enzyme immunoassay (EIA), an enzyme is covalently conjugated with one component of a specifically binding antigen-antibody pair, and the resulting enzyme conjugate is reacted with a substrate to produce a signal which is detected and measured. The signal may be a color change, detected with the naked eye or by spectrophotometric technique, or may be conversion of the substrate to a product detected by fluorescence. Conventional EIA may be performed by either competitive or sandwich mode, as disclosed by Schuurs et al. in U.S. Patent No. 3,654,090.

In other situations, assay for an enzyme itself may be indicated. In fact, enzyme assays may account for as much as 25% of the total work load in larger hospital laboratories, and as many as 20-25 enzymes may be routinely assayed. Detailed procedures for these routine assays may be found in any standard textbook on clinical or diagnostic chemistry.

In an enzyme assay, enzyme concentration in a body fluid, such as serum, urine and cerebrospinal fluid, is determined to be either within a normal range or outside of the normal range. In some assays; an abnormal enzyme concentration is the result of a bacterial or viral infection, and assay for a specific enzyme is diagnostic for a specific pathogen. In other cases, mere detection of an enzyme not normally present in a fluid, such as serum, may be indicative of tissue or organ damage. As with EIA, assay for an enzyme depends on catalysis by the enzyme of a substrate to a product providing a signal which is detected or measured.

Esterases are often used as labels in EIA. Commonly used substrates are indoxyl esters and esters of nitrophenols. Kramer et al., in Journal of Biological Chemistry, 235, 1785, (1960) disclose that 2,6-dichloroindophenyl acetate, 3′,5′-dichloroindophenyl acetate and 3′,5′-dibromoindophenyl butyrate are hydrolyzed by cholinesterase but are not hydrolyzed by acetylcholinesterase. The authors state that, "It is not unexpected that the 3′,5′ disubstituted substrates are not hydrolyzed by acetylcholinesterase."'

U.S. Patent No. 3,515,644 to Kramer et al. discloses a method for detection of anticholinesterase compounds which depend on inhibition of hydrolysis of 3′,5′-dichloroindophenylacetate by cholinesterase.

Barendsz, in the International Journal of Environmental Analytical Chemistry, 6, 89 (1979) discloses that butyrylcholinesterase hydrolyzes 1,6-dichloroindophenyl acetate.

Chiu et al. in Life Sciences, 9, 465 (1970) report that acetyl cholinesterase hydrolyzes both 2,6-dichloroindophenyl acetate and butyrate, in contrast to the data of Kramer et al.

In any enzyme assay, the chief requirement of a substrate is to provide a sensitive detection method for the enzyme. Most conventional chromogenic substrates are initially colorless and give a color on reaction with the enzyme. Ideally, the substrate should give completely soluble products with a high extinction coefficient (i.e. dense color per unit degraded). The substrate should also be cheap, safe and easy to use.

## SUMMARY OF THE INVENTION

One aspect of the invention is a chromogenic substrate for an esterase, 4-[(3,5-dichloro-4-butyryloxyphenyl)imino]-2,5-cyclohexadien-1-one, (3′,5′-dichloroindophenyl butyrate) hereinafter referred to as DCIPB.

In another aspect of the invention, the substrate may be used in an immunoassay for a ligand. A preferred assay is an EIA in which an antiligand is affixed to a solid support and contacted with a tracer which includes an esterase, preferably a carboxyesterase, conjugated to either the ligand or a second antiligand. After binding of antiligand, ligand and tracer, the solid phase is contacted with DCIPB. The DCIPB is cleaved by the esterase to 2,6-dichloroindophenol, and the color is measured.

The immunoassay of the invention may be configured as a dual enzyme assay in which a hydrolase component of the tracer removes a blocking group from a blocked inhibitor to release free inhibitor into the assay medium. Free inhibitor, which is proportional to ligand concentration, inhibits the conversion of DCIPB to 2,6-dichloroindophenol by an esterase.

Another embodiment of the invention is assay for a hydrolase which, in this aspect of the invention is referred to as the unknown enzyme, suspected of being present in a liquid. The hydrolase may be assayed by the dual enzyme assay described above in which the hydrolase removes a blocking group from a blocked inhibitor, and the resulting free inhibitor inhibits an esterase from converting DCIPB to 2,6-dichloro indophenol.

A third aspect of the invention is a kit of materials including DCIPB for performing an assay for a ligand or an enzyme.

Thus, the invention provides a substrate for an esterase, preferably a carboxyesterase, which has greater stability than conventional alternative substrates, such as esters of indoxyl. The product from interaction of the substrate and the esterase, 2,6-dichloroindophenol, is soluble in aqueous solution, deep blue in color, having an absorption maximum at 620 nm, and a relatively high extinction coefficient ($\epsilon$ = 1.8 x $10^4$ $m^{-1}$ $cm^{-1}$). These properties make it superior to alternative substrates in detection of liver esterases, whether in assay for a hydrolase or in an EIA for a ligand.

## BRIEF DESCRIPTION OF THE DRAWINGS

Fig. 1 illustrates the results of a typical sandwich assay for an antigen using the substrate of the invention;

Fig. 2 illustrates the results of a dual enzyme assay for an antigen using the substrate of the invention; and

Fig. 3 illustrates the results of a dual enzyme assay for a hydrolase using the substrate of the invention.

## DETAILED DESCRIPTION

While this invention is satisfied by embodiments in many different forms, there is described in detail preferred embodiments of the invention, with the understanding that the present disclosure is to be considered as exemplary of the principles of the invention and is not intended to limit the invention to the embodiments described. The scope of the invention will be measured by the appended claims and their equivalents.

The chromogenic substrate of the invention, DCIPB, may be prepared from 2,6-dichloroindophenol in accordance with the procedure of Example 1.

An enzyme-linked immunosorbent assay (ELISA), either sandwich or competitive, may be performed using carboxyesterase as the enzyme and DCIPB as the enzyme substrate. Alternatively, a dual enzyme assay in which two or more sequential enzyme reactions may be performed to amplify the assay signal, the terminal enzyme reaction being catalysis of the conversion of DCIPB to 2,6-dichloroindophenol by carboxyesterase. In the dual enzyme configuration, assay sensitivity is enhanced by including in the assay medium a particular blocked inhibitor of carboxyesterase.

An immunological reaction is used in the method of the invention for detection of the ligand in the unknown sample. By the term "immunological reaction," as used herein, is meant a specific binding reaction of an antigen and an antibody, a hapten and an antibody, or any appropriate analogue of an

antigen, an antibody, or a hapten which also binds specifically.

The immunological reaction may be carried out in any suitable liquid. For example, the liquid may be a body fluid suspected of containing the ligand, such as serum, urine, cerebrospinal fluid, pleural fluid or the like. Alternatively, the liquid may be water, saline or any appropriate buffer, or a mixture of body fluids and other liquids to which has been added a sample suspected of containing ligand.

In a preferred embodiment of the invention, one or more assay components may be attached to the surface of a solid support. As known in the art, the solid support may be any support which does not substantially interfere with the assay. Exemplary of solid supports which may be used are glass and polymeric materials, such as polyethylene, polyvinylidene fluoride, polystyrene and the like. Such supports may be fabricated into any suitable shape, such as sheets, tubes, wells, membranes or plates, such as microtiter plates. For example, an assay component may be attached to the inside walls and bottom of a tube, preferably a plastic tube with one closed end or to the wells of a microtiter plate; or to a membrane, such as a nylon or nitrocellulose membrane. Preferably, after the desired quantity of antiligand is attached to the solid support, any remaining binding sites on the support may be filled by an inert protein. The inert protein may be any protein, as, for example, ovalbumin, which can be attached to the support and which does not interfere in any way with the specific binding reactions between the ligand, antiligand and tracer, as described below.

Preferably, the antiligand attached to the solid support is incubated with ligand and tracer to bind both to the solid support. After a wash step to remove interfering materials, the remaining assay components may be added and the assay carried to completion as described below.

The ligand may be from any source, and may be an antigen, an antibody or a hapten. For example, the ligand may be an antigen present in a body fluid, or it may be isolated from a body fluid and subsequently introduced into a different liquid, such as buffer. In other cases, the ligand may be from a source other than a body fluid, as, for example, a culture of microorganisms or a cellular extract thereof. Preferred ligands are antigens, most preferably viral antigens present in a body fluid, such as Herpes simplex virus (HSV), Adenovirus, Influenza A virus, Parainfluenza 3 virus and Respiratory syncytial virus.

The antiligand is contacted with the ligand in the liquid to induce the immunological reaction. The antiligand may be an antigen or an antibody, either monoclonal or polyclonal, or it may be any appropriate analogue thereof which reacts specifically with the ligand. In addition, the antiligand may be an antibody complex consisting of a plurality of bound antibodies, as, for example, a second antibody bound specifically to a first antibody. Alternatively, the ligand may bind to several different antiligands, for example, an ensemble of polyclonal antibodies or a mixture of several monoclonal antibody molecules which bind simultaneously to different surface areas of the ligand. Generally, the second antibody is raised against the first antibody in a different species. The plurality of bound antibodies in the complex may contain from about two to ten or more antibodies. In the sandwich assay of the invention, it is preferred to use excess antiligand having sufficient binding sites to bind essentially all of the ligand.

In the ELISA of the invention, the enzyme portion of the tracer may be carboxyesterase conjugated to the ligand (competitive assay) or to the antiligand (sandwich assay). If the assay is a dual enzyme assay, the tracer comprises a first enzyme, described below, conjugated to the ligand (competitive assay) or to a second antiligand (sandwich assay) and the carboxyesterase serves as a second enzyme. Conjugating of enzymes to the ligand or antiligand is preferably by covalent linkage and is performed prior to the immunological reaction. Covalent conjugation of enzymes to ligands or antiligands is conventional and well known to those skilled in the art.

The first enzyme in the dual enzyme assay removes a blocking group from a blocked inhibitor. Suitable first enzymes are generally hydrolases, such as phosphatases, peptidases, esterases, glycosidases and the like. Exemplary of, but not limited to, suitable first enzymes are trypsin, thrombin, mammalian liver esterase; acetylcholinesterase, β-galactosidase, or most preferably, alkaline phosphatase.

The liquid containing the ligand, the antiligand and the tracer may be incubated, if necessary, to induce binding. Incubation may be carried out at any temperature and for any length of time suitable to facilitate binding, preferably from about 20° to 40° for about 1 minute to 4 hours. Antiligand, ligand and tracer which are bound are hereinafter referred to as the bound fraction and antiligand, ligand and tracer which do not bind are hereinafter referred to as the free fraction. The assay may, but need not, be carried out in such a way that equilibrium is established between the bound and free fractions.

The bound fraction may be separated from the free fraction in the liquid phase of the assay mixture by any conventional method, such as filtration, decantation, centrifugation, aspiration and the like. When the immunological reaction has been carried out on a solid support, the liquid phase is conveniently decanted and the solid support washed to ensure removal of the free fraction and any other materials which would interfere with the assay and resuspended in a suitable liquid such as water, saline or buffer. The blocked

inhibitor is then added, and the pH is adjusted to any level, preferably 6-8, which does not cause non-enzymatic removal of the blocking group, as described below.

The blocked inhibitor may be any material which may be converted by the first enzyme to an inhibitor of carboxyesterase. The preferred blocked inhibitor has two components, the inhibitor and the blocking group. The most preferred blocked inhibitor is a blocked inhibitor which is unreactive toward carboxyesterase until its blocking group is removed by the first enzyme and the inhibitor is liberated into the assay medium. The blocking group may preferably be one which can be covalently conjugated to the inhibitor by a bond which can be cleaved substantially selectively by the first enzyme, and the inhibitor component should preferably inhibit the activity of carboxyesterase while having substantially no effect on the first enzyme.

Carboxyesterase is the preferred enzyme in the ELISA of the invention, and is the preferred second enzyme in the dual enzyme assay of the invention. While the invention contemplates carboxyesterase from any source, it is preferred to use a liver carboxyesterase, most preferably mammalian liver carboxyesterase, such as or pig liver carboxyesterase, or, most preferably, rabbit liver carboxyesterase, RLE.

As mentioned above, the hydrolase component of the tracer cleaves the blocking group from the blocked inhibitor to provide the inhibitor of the carboxyesterase. Suitable inhibitors and blocked inhibitors are illustrated by the general formula I, set forth below, wherein the nature of group B, as described later, determines whether the compound is an inhibitor or a blocked inhibitor:

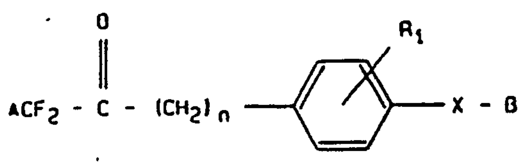

I

In formula I, $R_1$ may be H, lower alkyl of 1-6 carbon atoms, branched or unbranched, nitro, alkoxy, halogen and the like; X may be O, S or $NR_2$ wherein $R_2$ may be H or lower alkyl of 1-6 carbon atoms; n may be 1-6; A may be F or $CF_3$; and B may be H, a phosphoric acid or salt, a glycosyl group, an amino acid residue, such as a lysine or arginine residue covalently conjugated to X through the amino acid carboxyl group, an acyl group of 2-4 carbon atoms such as an acetyl or butyryl group, or a peptide of the formula II

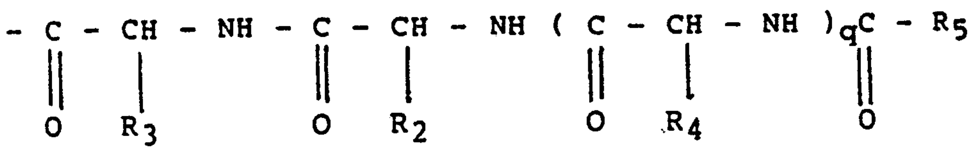

II

wherein $R_3$ is $(CH_2)_4NH_2$, $(CH_2)_3NH$ - 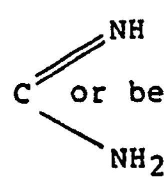 or benzyl

$R_4$ may be H, lower alkyl or hydroxy-lower alkyl of 1 to 4 carbon atoms, branched or unbranched, $CH_2COOH$ or $(CH_2)_2COOH$; $R_5$ may be lower alkyl or lower alkoxy of 1 to 4 carbon atoms, branched or unbranched, phenyl, or benzyloxy; and g may be 0-10.

When B is H, formula I represents enzyme inhibitors. When B is any group other than H, formula I represents blocked enzyme inhibitors. When B is a phosphoric acid or salt thereof, it is intended that B have the formula III

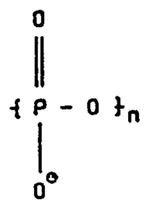

III

wherein P is bonded to X and n may be as described above.

The inhibitor and blocked inhibitor in accordance with formula I may be synthesized by any sequence of conventional chemical reactions as may be envisioned by one skilled in the art. Suitable and convenient methods are given in the Examples, below. The following list of effective enzyme inhibitors is intended to be exemplary only.

| Name | nmr data | Ki (M)* (Esterase) |
|---|---|---|
| 1. 1,1,1-trifluoro-3-(4-hydroxyphenyl)propanone | (CDCl3) - 3.91(s,2H), 5.21(bs,1H), 6.90(d,2H), 7.10(d,2H) | 2.0 x 10-6, RLE |
| 2. 1,1,1-trifluoro-3-(3-hydroxyphenyl)-2-propanone | (CDCl3) - 4.00(s,2H), 4.80(bs,1H), 6.80(m,3H), 7.30(m,1H) | > 10-4, PLE |
| 3. 1,1,1-trifluoro-4-(4-hydroxyphenyl)-2-butanone | (CDCl3) - 2.95(m,4H), 4.90(bs,1H), 6.92(dd,4H)J = 4.60Hz | 2.0 x 10-8, RLE |
| 4. 1,1,1-trifluoro-4-(3-hydroxyphenyl)-2-butanone | (CDCl3) - 2.94(t,2H), 3.05(t,2H), 6.70(bs,1H), 6.80(m,3H), 7.15(m1H) | 1.0 x 10-7, RLE |
| 5. 1,1,1-trifluoro-5-(4-hydroxyphenyl)-2-pentanone | (CDCl3) - 1.91(t,2H), 2.59(t,2H), 2.68(t,2H), 5.23(bs,1H), 6.95(d,2H), 7.10(d,2H) | 1.0 x 10-8, RLE |
| 6. 1,1,1-trifluoro-5-(3-hydroxyphenyl)-2-pentanone | (CDCl3) - 1.95(p,2H), 2.70(t,2H), 2.95(t,2H), 5.40(bs,1H), 6.70(m,3H), 7.30(m,1H) | 1.7 x 10-7, RLE |
| 7. 1,1,1-trifluoro-6-(4-hydroxyphenyl)-2-hexanone | (CDCl3) - 1.63(m,4H), 2.59(q,2H), 2.70(q.2H), 6.55(bs,1H), 6.77(d,2H) 7.02(d,2H) | 2.0 x 10-8, RLE |
| 8. 1,1,1,2,2-pentafluoro-5-(4-hydroxyphenyl)-3-pentanone | (CDCl3) - 2.94(m,2H), 3.04(m,2H), 4.75(bs,1H), 6.90(d,2H), 7.10(m,2H) | 8.0 x 10-7, RLE |

* PLE, Pig Liver Esterase (E.C. 3.1.1.1)
RLE, Rabbit Liver Esterase (E.C. 3.1.1.1)

As mentioned earlier, another embodiment of the invention is a competitive assay wherein the tracer is a predetermined quantity of the ligand conjugated to the first enzyme. In a competitive assay, the quantity of antiligand used is insufficient to bind all of the ligand and tracer present in the assay liquid so that ligand and tracer compete for the limited number of antiligand binding sites. Thus, in a competitive assay, the quantities of ligand and tracer which bind to the antiligand (bound fraction) are inversely proportioned to their concentrations in the assay liquid.

If additional signal amplification is desired, a multistage cascade amplification assay may be carried out wherein a plurality of reagents in the assay medium react sequentially leading ultimately to unblocking of the blocked inhibitor. In describing this embodiment of the invention, it is convenient to consider the first enzyme described above as a primary enzyme which enzymatically converts a reagent in the assay medium to a secondary enzyme which unblocks the blocked inhibitor for the above-described second enzyme. Further, the secondary enzyme, or any subsequent enzyme, may react with additional reagents to provide additional enzymes which may continue the cascade of enzymatic reactions until the blocked inhibitor is unblocked. By proper selection of reagents to be added to the assay medium, any desired number of amplification stages may be carried out.

It is evident that an almost unlimited number of competitive and sandwich assay configurations which fall within the scope of the invention can be envisioned. Further, the invention provides assay configurations

which are suitable for either detection of the ligand or determination of ligand concentration. Ligand concentration may be determined by comparing the magnitude of the signal generated with the unknown sample with the magnitude of the signal measured upon assay of a range of known quantities of the ligand assayed under essentially identical conditions. When the method of the invention is to be used for determination of ligand concentration, it is advantageous to read signal intensity by an appropriate instrument, such as a spectrophotometer, for example, a Beckman DU7 Spectrophotometer, Beckman Instruments, Inc., Irvine,: California.

In another embodiment of the invention, a dual enzyme assay which includes the blocked inhibitor described above may be used for determination of an unknown enzyme suspected to be present in a fluid. In this embodiment of the invention, the unknown enzyme corresponds to the first enzyme component of the tracer in the above-described dual enzyme assay. Accordingly, any unknown enzyme may be determined which is capable of removing the blocking group from the blocked inhibitor. Preferred unknown enzymes are hydrolases, as described above. Thus, all steps and reagents in assay for an unknown enzyme are the same as described for the dual enzyme assay except the assay does not include a ligand, antiligand and immunological reaction.

Another aspect of the invention is a reagent kit or package of materials for performing an assay for a ligand or an enzyme in accordance with the method of the invention. The kit may include an antiligand affixed to a solid support, an esterase, preferably carboxyesterase, and DCIPB. In one embodiment of the kit, the esterase may be conjugated to a second antiligand. In a second embodiment of the kit, the kit includes a blocked inhibitor of the esterase and a hydrolase may be conjugated to the second antiligand. The kit may also include standards for the ligand, as, for example, one or more ligand samples of known concentration, or it may include other reagents, enzyme substrates, or other labeled or unlabeled specific ligands, antiligands or complexes thereof useful in carrying out the assay. It may include solutions, such as saline or buffers. The components of the kit may be supplied in separate containers, as, for example, vials, or two or more of the components may be combined in a single container.

## EXPERIMENTAL

Routine Analytical Techniques - Flash Silica gel chromatography was performed on ICN silica gel 32-63 mesh at 3-7 psi. Analytical TLC was performed on 0.25 mm 5 x 20 cm aluminum-backed silica gel plates from EM Scientific. Preparative TLC was performed on 2.0 mm 20 x 20 cm glass-back silica gel plates from EM Scientific. Melting points were performed on a Thomas Hoover capillary melting point apparatus and are uncorrected. NMR spectra were recorded on an IBM WP-200SY spectrophotometer and chemical shifts are reported in ppm relative to trimethylsilane. HPLC was performed on a Waters 510 two pump system with detection using one of two solvent systems on a Brownlee AX- 300 7 x 250 mm column; System A) initial hold for 5 minutes at 30mM NH4OAc pH 6.5 followed by a linear gradient to 2.0- M NH4OAc over a 30 minute period followed by a hold at 1.0 M NH4OAc for 5 minutes. System B) used an isocratic buffer system of 30mM NH4OAc pH 6.5 for 40 minutes. Flow rates were 1.0 mL/minute. Gas chromatography was performed on a H.P. 5840A Gas Chromatograph equipped with a FID and an automatic injector using a 30 M DB-1 Megabore column purchased from J&W Scientific, Inc. GC conditions were as follows: A three minute hold at 100°C followed by a 10°C/minute gradient to 250°C followed by a 3.0 minute hold at 250°C at 16.0 mL/minute flow rate.

Inhibition constants were measured in 50 mM Tris pH = 8.0. Enzyme and inhibitor were incubated at ambient temperature for 20 minutes. Substrate for the enzyme was then added and the rate of hydrolysis was followed spectrophotometrically. The substrate for PLE and RLE was o-nitro-phenylbutrate and for AChE was acetyl thiocholine and Ellman's reagent.

The following examples are provided to further describe the invention but are not to be construed as limitative of the invention.

## EXAMPLE I

Preparation of 4-[(3,5-dichloro-4-butyryloxyphenyl)imino]-2,5-cyclohexadien-1-one (DCIPB)

A 1L round bottom flask was charged with 750 mL of acetonitrile and 30.0 g (0.103 mol) of 2,6-dichloroindophenol (sodium salt, hydrate). Butyric anhydride (25 mL, 0.153 mol) was combined with 10 mL of pyridine (0.124 mol) and 10 mL of acetonitrile in an addition funnel. This mixture was added dropwise to the well-stirred reaction mixture over a period of one hour. Stirring was continued for an additional 16 hours at ambient temperature, at which time the reaction mixture was concentrated by rotary evaporation under reduced pressure. The resulting syrup was diluted with 500 mL of ethyl acetate and washed with 250 mL of 1 N hydrochloric acid, 200 mL of saturated sodium bicarbonate, and dried over anhydrous magnesium sulfate. The crude product was concentrated to a syrup by rotary evaporation. Two isomers were present in the crude material because reaction with butyric anhydride can occur at either of the two reactive phenolate centers. These isomers were separated by flash chromatography on silica in ethyl acetate/hexane (15/85). Purity of the fractions was assessed by TLC in ethyl acetate/hexane (20/80). The desired compound has an Rf of 0.32, while the other isomer runs at 0.38. Fractions contaminated with the undesired isomer were rechromatographed. Pooled product was obtained in a 64% yield (22.2 g). NMR(CDCl3): 1.09(t,3H), 1.86-(m,2H), 2.67(t,2H), 6.66(m,2H), 6.90(s,2H), 7.07(d,1H), 7.25(d,1H).

## EXAMPLE II

Diammonium [4-(3-oxo-4,4,4-trifluorobutyl)phenyl]phosphate

A. Preparation of Ethyl 2-(4-methoxybenzyl)-3-oxo-4,4,4-trifluorobutanoate

A 1 L four neck round bottom flask, fitted with reflux condenser, dropping funnel, argon inlet, and magnetic stirrer was charged with 7.17 g (0.149 mol) of a 50% (w/v) oil dispersion of sodium hydride and 300 mL of dry ethyl ether. Absolute ethanol (9.0 mL) was slowly added to the stirred solution. After the evolution of hydrogen stopped, a mixture of 25 g (0.136 mol) of ethyl 4,4,4-trifluoroacetoacetate and 21.3 g (0.136 mol) of 4-methoxybenzyl chloride was added over a 1 hour period. The resulting mixture was refluxed overnight, cooled, extracted with water, 1 N hydrochloric acid, dried over anhydrous magnesium sulfate and rotary-evaporated under reduced pressure. The crude reaction mixture (33.5 g) was chromatographed on a 60 mm xx 300 mm silica gel column with ethyl acetate/hexane (25/75). Similar fractions were combined and gave 9.4 g (23%) of the (spectroscopically complex) product as an oil. NMR(CDCl3): 1.26-(m,3H), 3.77(s,3H), 4.12(m,2H), 7.08(m,2H).

B. Preparation of 1,1,1-trifluoro-4-(4-hydroxyphenyl)-butan-2-one

A 100 mL round bottom flask, fitted with reflux condenser, magnetic stirrer and argon inlet was charged with 2.05 g (6.7 mmol) of ethyl 2-(4-methoxybenzyl)-3-oxo-4,4,4-trifluorobutanoate (I), 20 mL of 31% (w/v) hydrogen bromide in acetic acid, and 10 mL of water. This mixture was heated overnight at 120° C, cooled, concentrated under reduced pressure and partitioned between dichloromethane and water. The organic layer was extracted sequentially with aqueous bisulfite, and saturated sodium bicarbonate, and then dried over anhydrous magnesium sulfate. Solvent was removed under reduced pressure. The crude reaction mixture was chromatographed on a 50 mm x 300 mm silica gel column with ethyl acetate/hexane (50/50). Similar fractions were combined and the solvent wash removed under reduced pressure to yield 600 mg (41%) as a clear oil. NMR(CDCl3): 2.95(m,4H), 5.40(bs, 1H), 6.93(dd,4H) J = 4, 60 Hz.

C. Preparation of diethyl [4-(3-oxo-4,4,4-trifluorobutyl)phenyl] phosphate

A 10 mL round bottom flask, fitted with argon inlet and magnetic stirrer was charged with 400 mg (1.8 mmol) of 1,1,1-trifluoro-4-(4-hydroxyyphenyl)butan-2-one, 400 mg (2.3 mmol) of diethyl chlorophosphate, 0.15 mL of dry pyridine and 5 mL of dichloromethane. The reaction mixture was stirred overnight at ambient temperature, filtered to remove pyridinium hydrochloride, extracted with 0.2 N hydrochloric acid, extracted with water, and dried over anhydrous magnesium sulfate. Solvent removal under reduced pressure afforded a crude yield of 600 mg of a brown oil. Two hundred mg (31%) of a clear oil was isolated from a

preparative TLC plate developed with ethyl acetate/hexane (50/50). NMR(CDCl3): 1.50(m6H), 3.0(m,4H), 4.20(m,4H), 7.15(s,4H).

D. Preparation of diammonium [4-(3-oxo-4,4,4-trifluorobutyl)phenyl] phosphate

A 25 mL one neck round bottom flask, fitted with argon inlet and magnetic stirrer was charged with 5.0 mL of dichloromethane, 140 mg (0.40 mmol) of diethyl [4-(3-oxo4,4,4-trifluorobutyl)phenyl] phosphate (III) and 2.0 mL of bromotrimethylsilane. After stirring this mixture for 3 hours at ambient temperature, 10 mL of methanol was added and the volatile materials were removed under reduced pressure. The residue was dissolved in water and adjusted to pH 7.0 with 1.0 N sodium hydroxide. The aqueous solution was extracted with diethyl ether and lyophilized to give 190 mg of a white solid. This material was dissolved in 10 mL of water, and purified by anion exchange HPLC. Gradient conditions: initial hold for 5 minutes at 20 mM ammonium acetate, pH 6.5; followed by a linear ramp to 1.0 M ammonium acetate over a 20 minute period; followed by a hold at 1.0 M ammonium acetate for 15 minutes. At a flow rate of 2.5 mL/min, the product eluted at approximately 32 minutes. Column capacity was 20 mg. Product fractions from several HPLC runs were pooled and lyophilized to yield 50 mg (37%). mp 235 - 240° C. NMR (D20): 1.90(m,2H), 2.56(m,2H), 4.65(s, DOH), 6.88(dd,4H) J =6, 82 Hz.

## EXAMPLE III

## Sandwich ELISA for Adenovirus

Preparation of antibodies:

Monoclonal antibodies against adenovirus were produced by conventional techniques, and originated from Balb/c mice immunized with purified hexon protein.

Preparation of antibody-porcine liver carboxyesterase conjugate:

Commercially available porcine liver esterase (PLE) (Sigma Chemical Co.) was exhaustively dialyzed against phosphate buffered normal saline solution, pH 7.6. This material was coupled to an anti-adenovirus monoclonal antibody using the "two-step" glutaraldehyde method: PLE, 2.0 mg, in 800 L of 50 mM phosphate, pH 7.2 was activated with 0.16% glutataraldehyde for 50 minutes at 20° C. To this was added 1.6 mg of antibody to give a final volume of 1.06 mL. After a 75 minute incubation at room temperature, the conjugate was isolated by size exclusion chromatography.

Construction of the immunological sandwich:

A polystyrene microwell plate (Nunc, Denmark) was coated for one hour at 37° C with 200 µL per well of a solution of 25 µg per mL anti-adenovirus monoclonal antibody in 10 mM sodium bicarbonate, pH 9.5. Following the incubation, the plate was rinsed twice with 200 µL per well of Tris buffered saline (10 mM tris-(hydroxymethyl)aminomethane, 150 mM sodium chloride, pH 7.5 (TBS)). At a volume of 150µ L per well, a sonicated suspension of adenovirus infected HeLa cells (antigen) was titered across the plate in three-fold serial dilutions. A similar titration was performed with uninfected (control) cells. The diluent for the antigen was TBS containing 0.2% casein and 0.05% polyoxyethylenesorbitan monolaurate (TWEEN-20). The plate was again incubated at 37° C for one hour and washed twice with TBS containing 0.2% casein. Anti-adenovirus antibody-porcine liver esterase conjugate was added to each well at an approximate concentration of 20 µg per mL (150 µL per well). Again, the plate was incubated for one hour at 37° C and washed three times with 200 µL per well with the TBS-casein buffer.

Generation of the signal:

The substrate, DCIPB, was prepared at a concentration of 0.18 mM in a solution composed of one part 50 mM diethanolamine, pH 9.0 mixed with one part 50 mM Tris, pH 7.5, 20% methanol. This solution was added to each well at a final volume of 300 µL and was allowed to incubate for five minutes at room temperature. A 50 µL per well addition of 1 mM 1,1,1-trifluoroacetophenone in 50 mM Tris, pH 7.5 was performed in order to quench the enzyme activity. The optical densities of each well were determined at 620 nm. The results are presented in Fig. 1.

## EXAMPLE IV

### Dual Enzyme Assay for Influenza A

Monoclonal antibodies against Influenza A were produced by conventional methods, and originated in mice immunized with Influenza A strain PR8/34.

Polyclonal antibodies against Influenza A were isolated from a goat immunized with a series of Influenza A strains: PR/8/34, A2/Taiwan, Victoria, and A2/Japan.

A polystyrene microtiter plate was coated with 2 µg/well of polyclonal antibody for one hour at 37° C in a solution of 10 mM carbonate, 20 mM ethylenediaminetetraacetate (EDTA), pH 9.5. The plate was washed three times with a wash buffer consisting of 2% (w/v) casein in TBS.

An antigen stock was prepared from Madin-Darby canine kidney (MDCK) cells infected with: Influenza A (WSN strain). The washed cells were added to a 10 mM Tris buffer containing 0.1% bovine serum albumin (BSA), 10 mM EDTA, 1 mM ethylene bis(oxyethelenenitrilo) tetraacetate (EGTA), 0.05% TWEEN-20, 0.1% phenol red, 0.01 mg/mL gentamicin. This antigen preparation was serially titered across the plate in two-fold steps. The plate was incubated as above to bind the antigen to the trapping antibody, washed with buffer, and incubated again with a tracer solution containing anti-Influenza monoclonal antibody conjugated to alkaline phosphatase diluted in wash buffer. Excess tracer was decanted, and the plate was washed three times with wash buffer.

A mixture of the blocked inhibitor from Example II ($1 \times 10^{-4}$M) and rabbit liver esterase (RLE) ($5 \times 10^{-9}$M) in 50 mM diethanolamine (DEOA), pH 9.0 was added to each well (150 µL), and incubated at room temperature for 20 minutes.

A solution of DCIPB (0.35 mM in 50 mM Tris, pH 7.5, containing 20% methanol) was added to each well (150 µL). After a five minute incubation, 40 µL of a solution of 1 mM 1,1,1-trifluoroacetophenone (TFAP) in 50 mM Tris, pH 7.5, was added to stop the reaction.

Control wells having no antigen typically showed intense color in ca 5 minutes. The addition of the commercially available esterase inhibitor 1,1,1-trifluoroacetophenone (TFAP) (Aldrich Chemical Co., Milwaukee, Wisconsin) stopped the color formation process. The same effect was achieved by the addition of free inhibitor (product B from Example II). After "stopping," test wells having a high concentration of antigen remained colorless for several hours.

The optical densities of each well were determined at 620 nm. Figure 2 shows the relationship of color formation to antigen concentration as determined with a Flow Laboratories Multiskan MC microplate reader. It is seen that the optical density (OD) of the solution in the control wells is substantially constant at about 1.0, and that the OD of the solution in the test wells is inversely proportional to antigen concentration and approaches the control value at very low antigen concentrations.

## EXAMPLE V

### Enzyme Detection By Duel Assay

To a microtiter plate was added 200 µL/well of 50 mM diethanolamine hydrochloride buffer, pH 9.0,

containing 0.5 mM MgCl and 0.02% (w/v) ovalbumin. Alkaline phosphatase was titered into these wells in five-fold serial dilutions. Fifty µL of mixture of rabbit liver carboxylesterase and blocked inhibitor was prepared in the diethanolamine buffer, and immediately added to each well. Final concentrations in each well were: 6.0 x $10^{-9}$ M esterase, 1.5 x $10^{-4}$ M blocked inhibitor. Following a 20 minute incubation at ambient temperature, 50 µL of a 2 mM solution of DCIPB in a solution composed of 300 mM Tris, pH 7.0 and methanol (40/60) was added to each well. Final concentrations in each well were: 3.3 x $10^{-4}$ M DCIPB, 10% methanol. Color development was allowed to proceed for three minutes at which time 50 µL of esterase stopping solution was added (1 mM 1,1,1-trifluoroacetophenone in 50 mM Tris, pH 7.5). The results were quantitated spectrophotometrically at 620 nm. (Fig. 3).

In summary, the invention provides a new chromogenic substrate and use of the new substrate in an assay method for detection or determination of a ligand present in a liquid sample at very low levels. The assay may be carried by ELISA or by a dual enzyme procedure in which a first enzyme removes a blocking group from an inhibitor of a second enzyme.

## Claims

1. A substrate for an esterase comprising 4-[3,5-dichloro-4-butyryloxyphenyl)imino]-2,5-cyclohexa dien-1-one.

2. A method for determining a ligand in a liquid comprising:

a) combining a first liquid suspected of containing a ligand with an antiligand affixed to a solid support and with a tracer, said tracer comprising a hydrolase, whereby said antiligand binds to said ligand and said tracer binds to one of said ligand and said antiligand, to give a bound phase on said support;

b) separating said support from said first liquid;

c) contacting said support with a second liquid containing an esterase and a blocked fluoroketone whereby said hydrolase converts said blocked fluoroketone to a fluoroketone, said fluoroketone being released into said second liquid;

d) adding to said second liquid 4-[3,5-dichloro-4-butyryloxyphenyl)imino]-2,5-cyclohexadien-1-one whereby conversion of said 4-[(3,5-dichloro-4-butyryloxyphenyl)imino]-2,5-cyclohexadien-1-one to 2,6-dichloroindophenol by said esterase is inhibited by said fluoroketone; and

e) detecting said ligand by inhibition of color formation due to said 2,6-dichloroindophenol.

3. A method for the detection of an antigen in a liquid comprising:

a) combining a first liquid suspected of containing an antigen with first and second antibodies, said first antibody being affixed to a solid support and said second antibody having alkaline phosphatase conjugated thereto whereby said antigen binds to said first and second antibodies to give a bound phase on said support;

b) separating said support from said first liquid;

c) contacting said support with a second liquid containing carboxyesterase and a blocked inhibitor whereby said alkaline phosphatase converts said blocked inhibitor to an inhibitor of said carboxyesterase, said inhibitor being released into said second liquid;

d) adding 4-[(3,5-dichloro-4-butyryloxyphenyl)imino]-2,5-cyclohexadien-1-one to said second liquid, the conversion of which to 2,6-dichloroindophenol by said carboxyesterase is inhibited by said inhibitor; and

e) detecting said antigen by detecting inhibition of formation of color due to 2,6-dichloroindophenol in said second liquid.

4. A method for immunoassay of a ligand in a liquid comprising combining a liquid suspected of containing a ligand with an antiligand affixed to a solid support and a tracer including an esterase to cause binding between said ligand, antiligand and tracer to give a bound fraction, contacting said bound fraction with 4-[(3,5-dichloro-4-butyryloxyphenyl)imino]-2,5-cyclohexadien-1-one whereby said esterase hydrolyzes said 4-[(3,5-dichloro-4-butyryloxyphenyl)imino]-2,5-cyclohexadien-1-one to 2,6-dichloroindophenol and determining said ligand by a signal associated with the color of said 2,6-dichloroindophenol.

5. A method for determining a hydrolase in a liquid comprising:

a) combining a liquid suspected of containing a hydrolase with a carboxyesterase and a blocked inhibitor, said hydrolase converting said blocked inhibitor to an inhibitor of said carboxyesterase, thereby releasing said inhibitor into said liquid;

b) adding to said liquid 4-[(3,5-dichloro-4-butyryloxyphenyl)imino]2,5-cyclohexadien-1-one, said carboxyesterase being capable of converting said 4-[(3,5-dichloro-4-butyryloxyphenyl)imino]-2,5-cyclohexa dien-1-one to 2,6-dichloroindophenol, said inhibitor inhibiting said carboxyesterase; and

c) determining said unknown enzyme by inhibition of color formation due to said 2,6-dichloroindophenol.

6. A kit of materials for performing an assay for a ligand in a liquid comprising an antiligand attached to a solid support, an esterase and 4-[(3,5-dichloro-4-butyryloxyphenyl)imino]-2,5-cyclohexadien-1-one.

7. The kit in accordance with Claim 6 further comprising a blocked inhibitor for said esterase and a hydrolase conjugated to a second antiligand.

8. The kit in accordance with Claim 6 wherein said esterase is conjugated to one of said ligand and a second antiligand.

9. The kit in accordance with Claim 6 further comprising at least one liquid containing ligand of known concentration.

10. A kit of materials for performing an assay for a hydrolase comprising a carboxyesterase, a blocked inhibitor for said carboxyesterase and 4-[(3,5-dichloro-4-butyryloxyphenyl)imino]-2,5-cyclohexadien-1-one to serve as a substrate for said carboxyesterase.

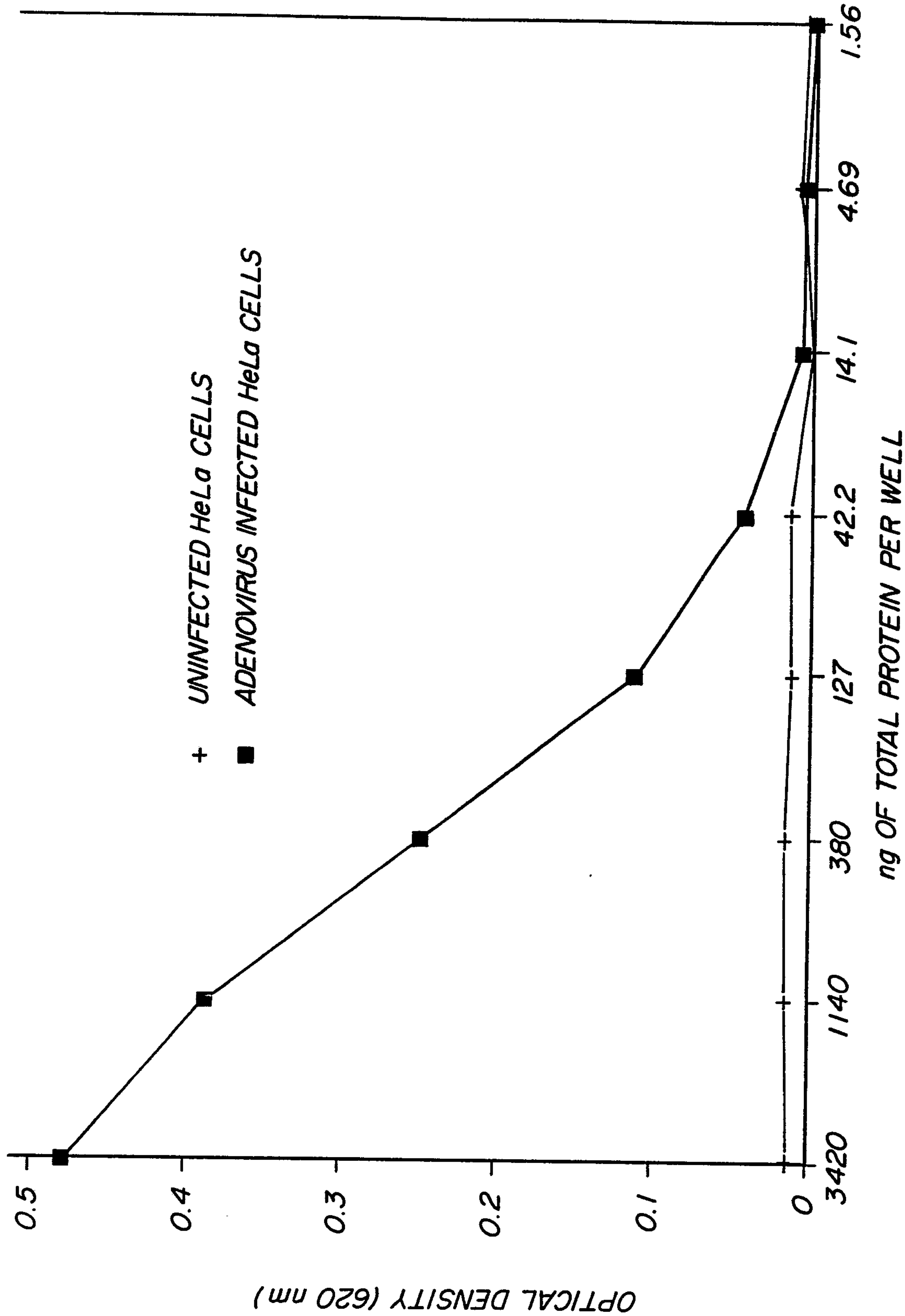

+ UNINFECTED HeLa CELLS
■ ADENOVIRUS INFECTED HeLa CELLS
OPTICAL DENSITY (620 nm)
0.5
0.4
0.3
0.2
0.1
0
3420
1140
380
127
42.2
14.1
4.69
1.56
ng OF TOTAL PROTEIN PER WELL


FIG-1

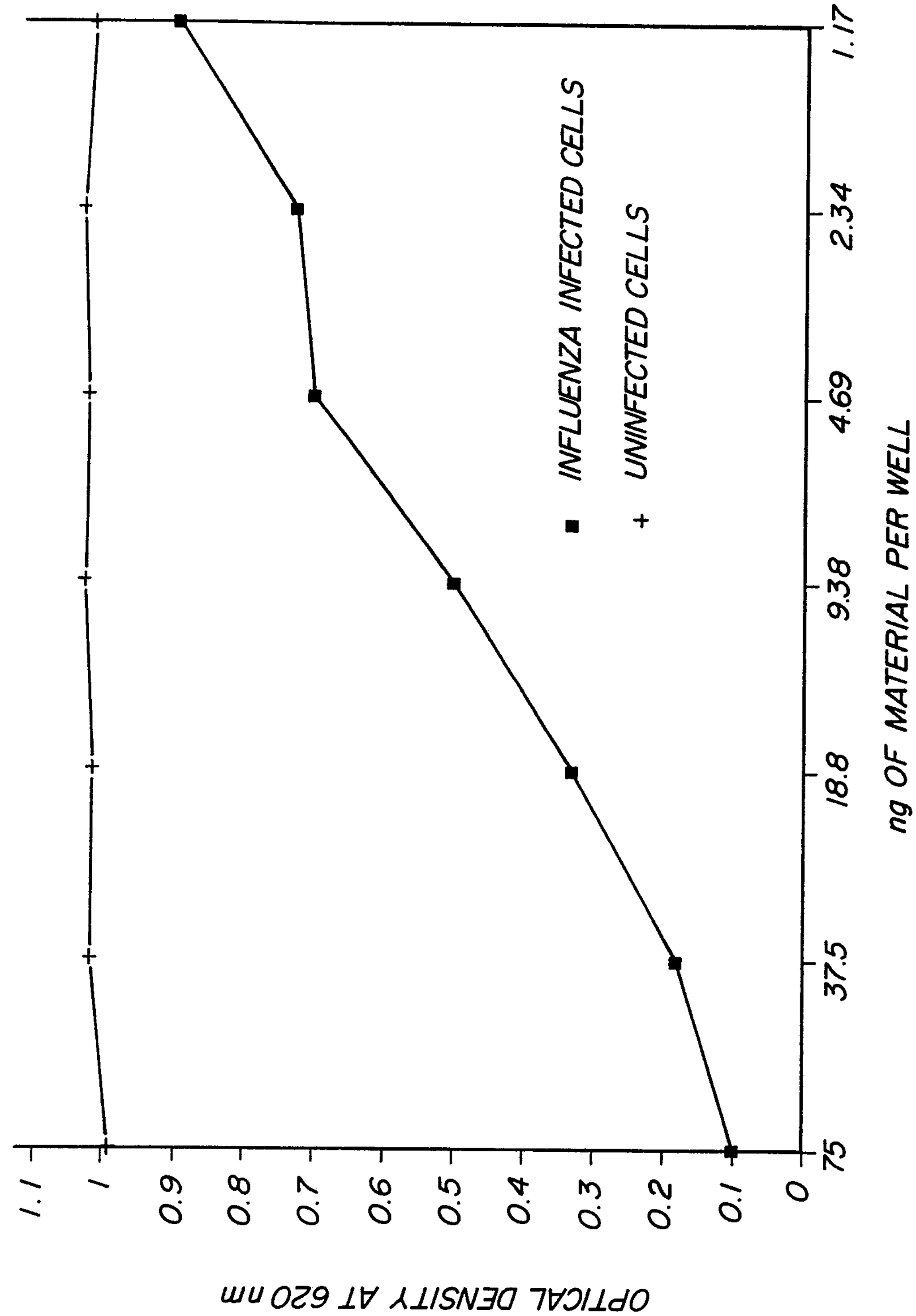
INFLUENZA INFECTED CELLS
UNINFECTED CELLS
OPTICAL DENSITY AT 620 nm
1.1
1
0.9
0.8
0.7
0.6
0.5
0.4
0.3
0.2
0.1
0
75
37.5
18.8
9.38
4.69
2.34
1.17
ng OF MATERIAL PER WELL

FIG-2

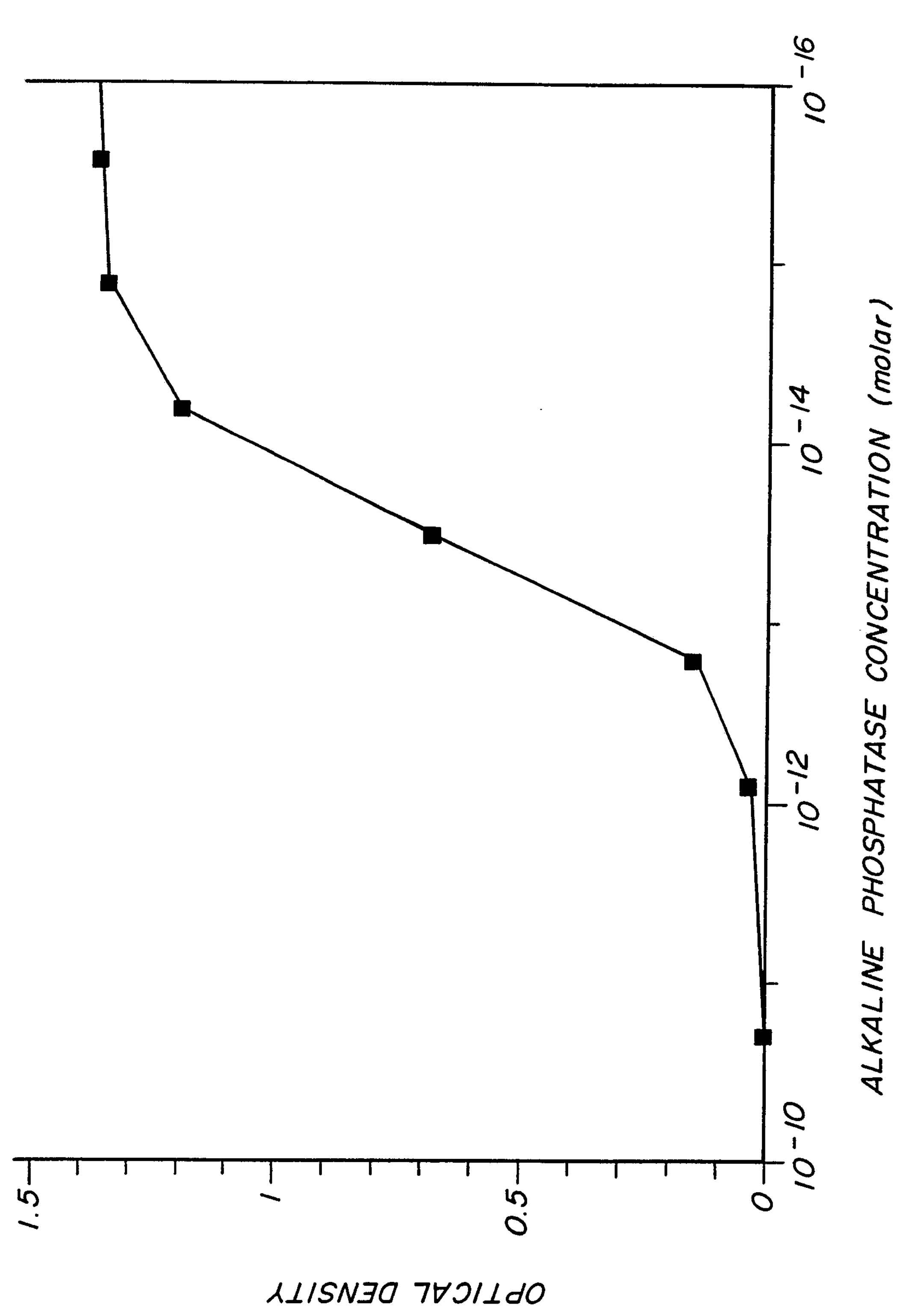
OPTICAL DENSITY
0
0.5
1
1.5
ALKALINE PHOSPHATASE CONCENTRATION (molar)
$10^{-10}$
$10^{-12}$
$10^{-14}$
$10^{-16}$

FIG-3